# EUROPEAN PATENT APPLICATION

(11) **EP 3 272 873 A1**
(43) Date of publication of application: **24.01.2018**
(21) Application number: 16768601.3
(22) Date of filing: 17.03.2016
(51) Int. Cl.: C12P 7/06, B01J 23/89, C01B 31/18, C12M 1/00

(54) **METHOD AND DEVICE FOR PRODUCING ORGANIC SUBSTANCE**

(30) Priority: 20.03.2015 JP 2015057556
(71) Applicant: Sekisui Chemical Co., Ltd., Osaka-shi, Osaka 530-8565 (JP)
(72) Inventor: SATOU, Kanetomo, Tsukuba-shi Ibaraki 300-4292 (JP); DASANAYAKE ALUTHGE, Rasika, Tsukuba-shi Ibaraki 300-4292 (JP)
(74) Representative: Merkle, Gebhard
(86) International application number: PCT/JP2016/058431
(87) International publication number: WO 2016/152698

(57) **Abstract**

It is an object of the present invention to convert CO₂ to CO by bringing about a reverse shift reaction in a relatively low-temperature condition, and thereby enhancing efficiency of generating organic substances such as ethanol.

A raw material gas g1 containing CO₂ and H₂ is subjected to a reverse shift reaction in a reverse shift reactor 12. Organic substances are generated from a raw material gas g2 after the reverse shift reaction in an organic substance generator 13. In the subjecting step, the raw material gas g1 is brought into contact with a reverse shift reaction catalyst 20. The reverse shift reaction catalyst 20 includes a support 21 and a catalyst metal 22 supported by the support 21. The catalyst metal 22 includes a transition metal. The catalyst metal 22 is preferably composed of Fe with at least one kind of metal from among Al, Ga, In, Cu, Ag, Au, Pd and Mn added thereto.

## Description

### Field of the Invention

The present invention relates to a method and an apparatus for producing organic substances, and particularly relates to a production method and a production apparatus suitable for producing ethanol from a raw material gas such as a synthetic gas.

### Background of the Invention

Plants that produce ethanol from a raw material gas such as a synthetic gas are well known (refer to Patent Documents 1 to 3). Patent Documents 1 to 3 disclose producing ethanol from a synthetic gas by a fermentation action of a certain type of anaerobic microorganisms.

Patent Document 4 discloses producing CO and H₂O from CO₂ and H₂ by a reverse shift reaction using a reverse shift reaction catalyst (formula (1)):

CO₂ + H₂ →CO + H₂O (1)

The reverse shift reaction catalyst contains an alkali earth metal carbonates of Ca, Sr or Ba and a complex oxide of Ca, Sr or Ba and Ti, Al, Zr, Fe, W or Mo. A temperature condition for the reverse shift reaction is 700 degrees C or higher.

### Prior Art Documents

### Patent Documents

Patent Document 1: Japanese Patent Application Publication No. 2004-504058
Patent Document 2: International Patent Application Publication No. WO2011/087380
Patent Document 3: United States Patent Application Publication No. US2013/0065282A1
Patent Document 4: Japanese Patent Application Publication No. 2010-194534

### Summary of the Invention

### Problem to be Solved by the Invention

According to the knowledge of the inverters, the anaerobic microorganisms mentioned above intake more CO than H₂ and CO₂ for fermentation. Therefore, a generation efficiency of ethanol can be enhanced by subjecting the H₂ and CO₂ in the raw material gas to a reverse shift reaction, and thereby converting CO₂ into CO. On the other hand, in a case of Patent Document 2, an operating cost may be high for the reverse shift reaction because it is required to make a temperature 700 degrees C or higher.

In view of the above, it is an objective of the present invention to make it possible for a raw material gas to perform the reverse shift reaction under low temperature conditions, and thereby reducing the operating cost and enhancing efficiency of generating organic substances such as ethanol.

### Means for Solving the Problems

To solve the problems mentioned above, a method of the present invention provides a method for producing organic substances from a raw material gas containing CO₂ and H₂, the method including steps of: subjecting the raw material gas to a reverse shift reaction; and generating organic substances from the raw material gas after the reverse shift reaction, wherein: the raw material gas is contacted with a reverse shift reaction catalyst in the subjecting step; the reverse shift reaction catalyst includes a support and a catalyst metal supported by the support; and the catalyst metal includes a transition metal.

An apparatus of the present invention provides an organic substance producing apparatus that produces organic substances from a raw material gas containing CO₂ and H₂, the apparatus including: a reverse shift reactor subjecting the raw material gas to a reverse shift reaction; and an organic substance generator generating the organic substances from the raw material gas after the reverse shift reaction, wherein: the reverse shift reactor includes a reverse shift reaction catalyst contactable with the raw material gas; the reverse shift reaction catalyst includes a support and a catalyst metal supported by the support; and the catalyst metal includes a transition metal.

By the reverse shift reaction, the CO₂ in the raw material gas can be converted into CO. By using the reverse shift reaction catalyst, conversion efficiency can be sufficiently enhanced. Moreover, a temperature condition can be set at 150 to 500 degrees C, which is lower than the temperature condition (700 degrees C or higher) of Patent Document 4 mentioned above. Accordingly, smaller energy is required for the reverse shift reaction, and an operation cost can be reduced. An efficiency of generating organic substances such as ethanol can be enhanced by making the raw material gas CO rich by the reverse shift reaction.

The transition metal may be Fe, Sc, Ti, V, Cr, Mn, Co, Ni, Cu, Zn, Y, Zr, Nb, Mo, Tc, Ru, Rh, Pd, Ag, Cd, La, Ce, Pr, Nd, Pm, Sm, Eu, Gd, Tb, Dy, Ho, Er, Tm, Yb, Lu, Hf, Ta, W, Re, Os, Ir, Pt, Au or Hg, for example.

Preferably, the catalyst metal includes Fe with at least one kind of metal selected from a group of Al, Ga, In, Cu, Ag, Au, Pd, and Mn added thereto.

Preferably, the catalyst metal includes Fe with Pd added thereto.

By this arrangement, the conversion efficiency of the reverse shift reaction can be further enhanced.

Preferably, the support may be composed of at least one selected from a group of SiO₂, CeO₂, TiO₂, Al₂O₃, MgO, ZrO₂, ZSM-5 (zeolite).

Preferably, an offgas generated in the generating step is mixed with the raw material gas before the reverse shift reaction.

Preferably, the apparatus further includes an offgas passage adapted to send out an offgas from the organic substance generator therethrough, the offgas passage extending to the reverse shift reactor.

By this arrangement, CO₂ generated at the time of producing the organic substances can be subjected to the reverse shift reaction, thereby being converted into CO and used for the production of the organic substances. Thus, CO₂ and CO can be used in a cyclic manner.

Preferably, the generating step includes a step of culturing microorganisms in a liquid culture medium; and the microorganisms fermentatively generate the organic substances from the raw material gas after the reverse shift reaction.

Preferably, the organic substance generator includes a culture tank adapted to culture microorganisms in a liquid culture medium therein; and the microorganisms fermentatively generate the organic substances from the raw material gas after the reverse shift reaction.

The efficiency of fermentative generation of the organic substances by the microorganisms can be enhanced by increasing a concentration of CO by the reverse shift reaction.

### Advantageous Effects of the Invention

According to the present invention, the raw material gas can be subjected to the reverse shift reaction under a relatively low temperature condition, and thereby the operation cost can be reduced. By converting CO₂ of the raw material gas into CO by the reverse shift reaction, the efficiency of generating the organic substances such as ethanol can be enhanced.

### Brief Description of the Drawings

FIG. 1 is a block diagram schematically showing an organic substance producing system according to a first embodiment of the present invention.
FIG. 2 is a block diagram schematically showing an organic substance producing system according to a second embodiment of the present invention.

### Mode for Carrying out the Invention

Embodiments of the present invention will be described hereinafter with reference to the drawings.

### <First Embodiment>

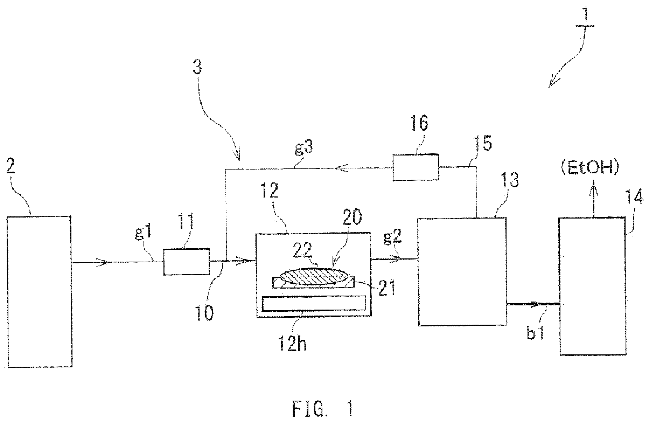

FIG. 1 shows a first embodiment of the present invention. An organic substance producing system 1 includes a raw material gas generator 2 and an organic substance producing apparatus 3. A raw material gas g1 is generated at the raw material gas generator 2. Organic substances (target substances) are generated from the raw material gas g1 by the organic substance producing apparatus 3. The raw material gas g1 is a synthetic gas (syngas). The target substance may be ethanol (C₂H5OH), for example.

The raw material gas generator 2 is a waste disposal facility in this embodiment. Wastes may include municipal wastes, tires, biomass, wooden chips and plastic wastes. The waste disposal facility 2 is provided with a melting furnace. In the melting furnace, the wastes are burnt by a highly-concentrated oxygen gas and decomposed at a low-molecular level. Eventually, the raw material gas g1 (synthetic gas) is generated.

The raw material gas g1 (synthetic gas) may contain CO, H₂ and CO₂ as major constituents. A constituent ratio may be around 30 vol % of CO, around 30 vol % of H₂ and around 30 vol % of CO₂, but it is not required that the constituent ratio should be as given above. Most of the remaining constituents of the raw material gas g1 may be N₂. The raw material gas g1 may further include minute amount of impure constituents such as H₂S, O₂ and benzene.

As shown in FIG. 1, the organic substance producing apparatus 3 includes a gas supply passage 10, a gas purifier 11, a reverse shift reactor 12 and an organic substance generator 13. The gas supply passage 10 extends from the waste disposal facility 2 to the organic substance generator 13. The gas purifier 11 and the reverse shift reactor 12 are disposed at a point along the gas supply passage 10. The gas purifier 11 includes a desulfurizing portion, deoxidizing portion and a debenzenizing portion.

The reverse shift reactor 12 is disposed at a point of the gas supply passage 10 on a downstream side with respect to the gas purifier 11. A reverse shirt reaction catalyst 20 is received in the reverse shift reactor 12. The reverse shift reaction catalyst 20 includes a support 21 and a catalyst metal 22. The support 21 may be composed of SiO₂, CeO₂, TiO₂, Al₂O₃, MgO, ZrO₂ or ZSM-5. In this embodiment, the support 21 is composed of silicon oxide (SiO₂). The catalyst metal 22 is supported by the support 21. The supported catalyst may be prepared by wet impregnation, co-precipitation, Schlenk Line, or the like.

The catalyst metal 22 may include a transition metal such as Fe.

Preferably, the catalyst metal 22 may include Fe with at least one kind of metal from among Al, Ga, In, Cu, Ag, Au, Pd, and Mn added thereto. One kind of metal may be added or two or more kinds of metal may be added.

More preferably, the catalyst metal 22 is composed of Fe with Pd added thereto.

A content rate of the catalyst metal 22 in the reverse shift reaction catalyst 20 may be preferably 5 wt % to 50 wt % of the entire reverse shift reaction catalyst 20 and more preferably around 20 wt of the entire reverse shift reaction catalyst 20.

A content rate of added constituents (Al, Ga, In, Cu, Ag, Au, Pd or Mn) in the catalyst metal 22 may be preferably 0.1 wt % to 1.0 wt % of the entire catalyst metal 22 and more preferably around 0.2 wt % of the entire catalyst metal 22.

A heater 12h (temperature controller) is disposed in the reverse shift reactor 12. A temperature of an inside of the reverse shift reactor 12, and thereby a temperature of the reverse shift reaction catalyst 20 can be controlled to be a desired temperature by the heater 12h.

The organic substance generator 13 is disposed subsequent to the reverse shift reactor 12. The organic substance generator 13 is provided with a culture tank. A liquid culture medium is stored in the culture tank. Gas-utilizing microorganisms are cultured in the liquid culture medium. Anaerobic bacteria disclosed in the Patent Documents 1 to 3 mentioned above may be used as the gas-utilizing microorganisms, for example. The gas-utilizing microorganisms synthesize ethanol (C₂H₅OH), etc. from CO and H₂, etc. by a fermentative action thereof.

A refiner 14 is disposed subsequent to the organic substance generator 13. The refiner 14 is composed of a distillation tower.

An offgas passage 15 extends from the organic substance generator 13. An offgas purifier 16 is disposed at a point along the offgas passage 15. The offgas purifier 16 includes a desulfurizing portion and a water eliminating portion. A downstream end of the offgas passage 15 is joined to the gas supply passage 10 at a point between the gas purifier 11 and the reverse shift reactor 12.

Ethanol (organic substance) is generated by the organic substance producing system 1 in the following manner:

### <Raw Material Gas Generating Step>

The raw material gas g1 is generated by burning wastes at the waste disposal facility 2.

The raw material gas g1 is introduced to the gas supply passage 10.

### <Raw Material Gas Purifying Step>

Impure components such as H₂S, O₂ and benzene in the raw material gas g1 are removed in the gas purifier 11. The impure components may be removed by using a catalyst. The impure components may be removed by condensation. Thereby, the raw material gas g1 is purified.

### <Reverse Shift Reaction Step>

Subsequently, the raw material gas g1 is introduced to the reverse shift reactor 12.

A temperature of the reverse shift reactor 12 is controlled to be 150 degrees C to 500 degrees C by the heater 12h.

A pressure of the reverse shift reactor 12 may be set at 0.8 to 2 atmospheric pressure (gauge pressure), for example.

The raw material gas g1 is contacted with the reverse shift reaction catalyst 20 in the reverse shift reactor 12. Thereby, at least a portion of CO₂ and H2 in the raw material gas g1 is subjected to a reverse shift reaction as expressed in the following formula (1):

CO₂ + H₂ →CO + H₂O (1)

Accordingly, CO₂ in the raw material gas g1 can be converted into CO. The raw material gas after the reverse shift reaction is referred to as "raw material gas g2" hereinafter. CO concentration is higher in the raw material gas g2 than in the raw material gas g1 before the reverse shift reaction. CO₂ concentration and H₂ concentration are lower in the raw material gas g2 than in the raw material gas g1.

By using the reverse shift reaction catalyst 20 having a structure and composition mentioned above as a catalyst, a conversion efficiency of CO₂ →CO can be sufficiently enhanced. The conversion efficiency of 20% or higher can be achieved, for example.

By using Fe with a minute amount of Pb added thereto as the catalyst metal 22, the conversion efficiency can be enhanced to around 50 %.

Thereby, the CO concentration in the raw material gas g2 can be made sufficiently high.

Moreover, temperature condition of about 150 degrees C to 500 degrees C may be sufficient for the reverse shift reaction, which is much lower than the temperature condition (700 degrees C or higher) in the Patent Document 1 mentioned above. Accordingly, energy required for the heater 12h can be reduced, and therefore, an operation cost can be reduced.

### <Fermentation Step (Organic Substance Generating Step)>

The raw material gas g2 from the reverse shift reactor 12 is introduced into the liquid culture medium in the organic substance generator 13. Then the gas-utilizing microorganisms in the liquid culture medium take in CO and H₂ in the raw material gas g2 and perform fermentation, thereby generating the target substance, ethanol.

The gas-utilizing microorganisms of this kind perform fermentation taking in more H2 than CO. Therefore, the efficiency of generating ethanol can be enhanced by making the CO concentration of the raw material gas g2 high in the reverse shift reaction step.

### <Refining Step>

A portion b1 of the liquid culture medium in the organic substance generator 13 is taken out and sent out to the refiner 14 composed of a distillation tower. The liquid culture medium b1 is distilled in the refiner 14. Thereby, the ethanol (EtOH) can be refined.

An amount of the culture medium in the organic substance generator 13 is arranged to be maintained constant by newly replenishing the culture medium to the organic substance generator 13 in an amount corresponding to the sent out amount.

### <Exhaust Step>

CO₂ is generated as a by-product during the fermentation of ethanol mentioned above. Therefore, a large amount of CO₂ is contained in an offgas g3 from the organic substance generator 13. Besides CO₂, the offgas g3 contains CO, H₂, etc. of the raw material gas g2 that were not used for the fermentation. The offgas g3 is sent to the offgas purifier 16. Impure components of the offgas g3 such as H₂O and a minute amount of H₂S are removed in the offgas purifier 16.

### <Reusing Step>

After that, the offgas g3 is joined to the gas supply passage 10 via the offgas passage 15 and mixed with the raw material gas g1 in the gas supply passage 10. Thereby, CO₂ in the offgas g3 can be provided to the reverse shift reactor 12 together with the raw material gas g1 for the reverse shift reaction. In short, CO₂ generated in the fermentation step as the by-product can be converted into CO, and the CO can be sent to the organic substance generator 13 as a part of the raw material gas g2 and can be used for fermentation. Thereby, CO₂ and CO can be used in a cyclic manner in which the CO₂ and CO are repeatedly converted into each other.

Other embodiments of the present invention will be described hereinafter. Same reference numerals are used in the drawings to designate parts that correspond to those in foregoing embodiments and description thereof will be omitted.

### <Second Embodiment>

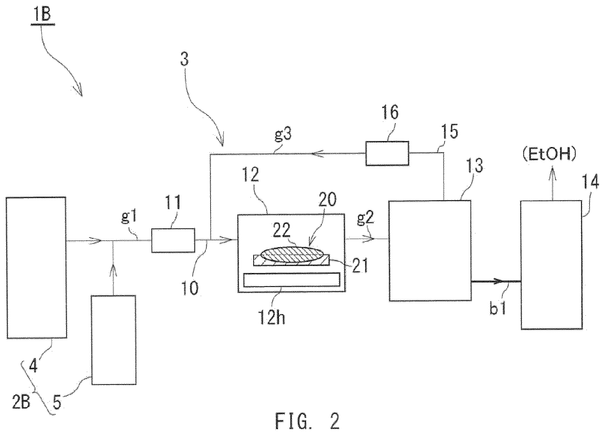

FIG. 2 shows a second embodiment of the present invention. A raw material gas generator 2B of an organic substance producing system 1B according to the second embodiment includes a carbon dioxide generator 4 and a hydrogen generator 5. The carbon dioxide generator 4 may be a coal power plant, a LNG power plant, a petroleum products manufacturing plant, a cement manufacturing plant, etc. In these plants, CO₂ is generated, but H₂ is hardly generated.

The hydrogen generator 5 is disposed at a point along a gas supply passage 10 from the carbon dioxide generator 4.

The hydrogen generator 5 may be a steam reforming plant for natural gas such as methane (CH₄). The methane is steam-reformed as expressed in the following formula (2):

CH₄ + H₂O →CO + 3H₂ (2)

The reformed gas (CO, H₂) is mixed with the CO₂ from the carbon dioxide generator 4. Thereby, a raw material gas g1 (synthetic gas) containing CO, H₂ and CO₂ can be obtained. The raw material gas g1 is supplied to a reverse shift reactor 12 via a gas purifier 11, and thereby subjected to a reverse shift reaction. Thus, a CO rich raw material gas g2 can be obtained. Thereby, an efficiency of generation of ethanol in an organic substrate generator 13 can be enhanced.

The present invention is not limited to the embodiments described above. Various modifications can be made without departing from the scope and spirit of the invention.

For example, the catalyst metal 22 of the reverse shift reaction catalyst 20 may be composed only of Fe, without containing the added constituents.

The organic substrate generator 13 may generate ethanol by bringing the raw material gas g2 into contact with the metal catalyst instead of by microbial fermentation.

### Explanation of Reference Numerals

- b1: culture medium
- g1: raw material gas
- g2: raw material gas after the reverse shift reaction
- g3: offgas
- 1: organic substance producing system
- 2: waste disposal facility (raw material gas generator)
- 2B: raw material gas generator
- 3: organic substance producing apparatus
- 4: carbon dioxide generator
- 5: hydrogen generator
- 10: gas supply passage
- 11: gas purifier
- 12: reverse shift reactor
- 12h: heater (temperature controller)
- 13: organic substance generator
- 14: refiner
- 15: offgas passage
- 16: offgas purifier
- 20: reverse shift reaction catalyst
- 21: support (silicon oxide substrate)
- 22: catalyst metal

## Claims

1. A method for producing organic substances from a raw material gas containing CO₂ and H₂, the method comprising steps of:
subjecting the raw material gas to a reverse shift reaction; and
generating organic substances from the raw material gas after the reverse shift reaction, wherein:
the raw material gas is contacted with a reverse shift reaction catalyst in the subjecting step;
the reverse shift reaction catalyst includes a support and a catalyst metal supported by the support; and
the catalyst metal includes a transition metal.

2. The method according to claim 1, wherein the catalyst metal includes Fe with at least one kind of metal selected from a group of Al, Ga, In, Cu, Ag, Au, Pd, and Mn added thereto.

3. The method according to one of claims 1 and 2, wherein the catalyst metal includes Fe with Pd added thereto.

4. The method according to any one of claims 1 to 3, wherein a temperature condition in the subjecting step is from 150 degrees C to 500 degrees C.

5. The method according to any one of claims 1 to 4, wherein an offgas generated in the generating step is mixed with the raw material gas before the reverse shift reaction.

6. The method according to any one of claims 1 to 5, wherein:
the generating step includes a step of culturing microorganisms in a liquid culture medium; and
the microorganisms fermentatively generate the organic substances from the raw material gas after the reverse shift reaction.

7. An organic substance producing apparatus that produces organic substances from a raw material gas containing CO₂ and H₂, the apparatus comprising:
a reverse shift reactor subjecting the raw material gas to a reverse shift reaction; and
an organic substance generator generating the organic substances from the raw material gas after the reverse shift reaction, wherein:
the reverse shift reactor includes a reverse shift reaction catalyst contactable with the raw material gas;
the reverse shift reaction catalyst includes a support and a catalyst metal supported by the support; and
the catalyst metal includes a transition metal.

8. The apparatus according to claim 7, wherein the catalyst metal includes Fe with at least one kind of metal selected from a group of Al, Ga, In, Cu, Ag, Au, Pd, and Mn added thereto.

9. The apparatus according to one of claims 7 and 8, wherein the catalyst metal includes Fe with Pd added thereto.

10. The apparatus according to any one of claims 7 to 9, wherein a temperature condition at the reverse shift reactor is from 150 degrees C to 500 degrees C.

11. The apparatus according to any one of claims 7 to 10, wherein the apparatus further includes an offgas passage adapted to send out an offgas from the organic substance generator therethrough, the offgas passage extending to the reverse shift reactor.

12. The apparatus according to any one of claims 7 to 11, wherein:
the organic substance generator includes a culture tank adapted to culture microorganisms in a liquid culture medium therein; and
the microorganisms fermentatively generate the organic substances from the raw material gas after the reverse shift reaction.
